Europäisches Patentamt

European Patent Office

Office européen des brevets

(19)

(11) Veröffentlichungsnummer: **0 216 179 B1**

# (12) EUROPÄISCHE PATENTSCHRIFT

(45) Veröffentlichungstag der Patentschrift: **21.08.91**

(51) Int. Cl.⁵: **C12Q 1/37, C12Q 1/56**

(21) Anmeldenummer: **86111844.6**

(22) Anmeldetag: **27.08.86**

(54) Verfahren zur Bestimmung von Proteaseinhibitoren.

(30) Priorität: **06.09.85 DE 3531778**

(43) Veröffentlichungstag der Anmeldung:
**01.04.87 Patentblatt 87/14**

(45) Bekanntmachung des Hinweises auf die
Patenterteilung:
**21.08.91 Patentblatt 91/34**

(84) Benannte Vertragsstaaten:
**AT BE CH DE FR GB IT LI LU NL SE**

(56) Entgegenhaltungen:
**EP-A- 0 048 989**
**EP-A- 0 168 738**

**PATENT ABSTRACTS OF JAPAN, Band 7, Nr.**
**195 (C-183)[1340], 25. August 1983; & JP - A -**
**58 98097 (DAIICHI KAGAKU YAKUHIN K.K.)**
**10.06.1983**

**PATENT ABSTRACTS OF JAPAN, Band 8, Nr.**
**93 (C-220)[1530], 27. April 1984; & JP - A - 59**
**11198 (DAIICHI KAGAKU YAKUHIN K.K.)**
**20.01.1984**

**ANNUAL REVIEW OF BIOCHEMISTRY, Band**
**49, 1980, Seiten 593-626, US; M. LASKOWSKI**
**et al.: "Protein inhibitors of proteinases"**

**EXPERIENTIA, Band 39, Nr. 4, April 1983, Sei-**
**ten 379-375, Basel, CH: P. FRIC et al.: " New**
**low-molecular inhibitors of pancreatic ela-**
**stase with possible in vivo application: Alky-**
**lamides of N-acylated tripeptides"**

**CLINICAL CHEMISTRY, Band 29, Nr. 2, Febru-**
**ar 1983, Seiten 225-236, Washington, US; J.**
**FAREED et al.: "Diagnostic efficacy of newer**
**synthetic-substrates methods for assessing**
**coagulation variables: A critical overview"**

(73) Patentinhaber: **BEHRINGWERKE Aktiengesell-**
**schaft**
**Postfach 1140**
**W-3550 Marburg 1(DE)**

(72) Erfinder: **Kolde, Hans-Jürgen, Dr.**
**Höhenweg 54**
**W-3550 Marburg(DE)**

(74) Vertreter: **Klein, Otto, Dr. et al**
**Hoechst AG Zentrale Patentabteilung Post-**
**fach 80 03 20**
**W-6230 Frankfurt am Main 80(DE)**

**Beschreibung**

Die Erfindung betrifft ein Verfahren zur Bestimmung von Proteaseinhibitoren mittels der entsprechenden Protease und einem Substrat für diese Protease.

Proteaseinhibitoren sind Proteine des Blutplasmas, die mit Proteasen reagieren und deren Aktivität regulieren. Die wichtigsten Proteaseinhibitoren im Blut sind alpha$_2$-Makroglobulin, alpha$_1$-Antitrypsin, Antithrombin III (AT III), alpha$_2$-Antiplasmin und C1-Esteraseinhibitor. Wie Proteasen haben auch Inhibitoren eine mehr oder minder hohe Spezifität. Die Verminderung der Aktivität von Proteaseinhibitoren kann bei Patienten zu lebensbedrohenden Situationen führen.

Bei solchen Patienten ist eine Bestimmung dieser Inhibitoren angezeigt, um therapeutische Maßnahmen rechtzeitig ergreifen zu können. Weiter ist während bestimmter Therapieformen und allgemein zum präoperativen "screening" von Patienten die Bestimmung von Proteaseinhibitoren eine allgemein durchgeführte diagnostische Maßnahme mit hohem Informationswert.

Zur Konzentrationsbestimmung von Proteaseinhibitoren werden immunologische Verfahren unter Verwendung von Nephelometrie oder Turbidimetrie, die radiale Immundiffusion, Enzym- oder Radioimmunoassays und ähnliche Methoden benutzt. Grundsätzlich werden jedoch bei allen bisher bekannten immunologischen Verfahren auch inaktive Moleküle von Proteaseinhibitoren und auch inaktive Enzym-Inhibitorkomplexe erfaßt. Es sind aber zahlreiche Fälle bekannt, in denen Proteaseinhibitoren in normaler Konzentration vorhanden sind, jedoch eine verminderte oder fehlende Aktivität aufweisen.

Daher werden diese Proteine zunehmend mit funktionellen Methoden bestimmt, die ihre Eigenschaft, Enzyme zu hemmen, ausnutzen. Üblicherweise wird das durch den zu bestimmenden Inhibitor hemmbare Enzym im Überschuß vorgelegt und nach Ende der Reaktion der Protease mit dem Inhibitor der Probe die Restaktivität des Enzyms bestimmt, beispielsweise mit einem chromogenen Substrat (Bestimmung von Antithrombin III: Thromb.Res. 5, 621-632, 1974).

Verschiedene Proteaseinhibitoren lassen sich so relativ einfach messen. Insbesondere durch Einsatz synthetischer Substrate wie Peptidparanitroaniliden oder ähnlicher Verbindungen, die sich fotometrisch oder fluorimetrisch bestimmen lassen, wurden die Empfindlichkeit, Präzision und Richtigkeit dieser Bestimmungen wesentlich verbessert. Dadurch konnten solche Proteine routinemäßig kinetisch oder mit Endpunktsmethoden gemessen werden.

Der Arbeitsablauf einer typischen Inhibitorbestimmung weist aber nach dem derzeitigen Stand der Technik noch Schwächen auf, die die Durchführung dieser Tests erschweren. Insbesondere können Inkubationszeiten von mehreren Minuten zur maximalen Hemmung des im Überschuß vorliegenden Enzyms nötig sein. Diese Inkubationszeiten müssen ziemlich genau eingehalten werden, da bei zu kurzer Inkubation die maximale Hemmung nicht erreicht wird, andererseits bei zu langer Inkubation eine zusätzliche Hemmung durch unspezifische Proteaseinhibitoren möglich ist. In einigen Fällen ist außerdem eine Vorverdünnung der Proben erforderlich. Weiterhin sind zur Bestimmung der restlichen Enzymaktivität relativ große Mengen von teuren chromogenen Substraten notwendig, um über eine Zeit von mindestens 3 Minuten eine lineare Kinetik zu erzielen. In Thromb.Res. 25, 245-253 (1982) ist beschrieben wie die Wechselwirkung eines synthetischen Substrates und Thrombin und dessen Inhibitor mit AT III in Gegenwart von Heparin gemessen werden kann. Es wurde eine konstante Menge an gereinigtem AT III mit verschiedenen Mengen an Substrat bzw. Heparin versetzt und aus der nichtlinearen Substrathydrolysekurve kinetische Daten ermittelt, die die Heparinwirkung beschreiben. Eine ähnliche Methode zur Bestimmung von Heparin ist beschrieben in Clin.Chem. 28, 1521-1524 (1982); jedoch ist hier die Analysenzeit recht lang.

Überraschenderweise wurde jedoch gefunden, daß in Plasma bei Konstanthalten der Substrat- und Proteasekonzentration und im Falle von AT III der Heparinkonzentration eine Aktivitätsbestimmung von Proteaseinhibitoren möglich ist, ohne daß man in der Probe mit Störungen durch Fibrinbildung oder wechselnde Fibrinogenkonzentrationten - das ja mit Thrombin reagiert - zu rechnen hat.

Gegenstand der Erfindung ist daher ein Verfahren zur Bestimmung eines Proteaseinhibitors mittels einer Protease, die durch diesen Inhibitor gehemmt werden kann, und eines Substrats für diese Protease, dadurch gekennzeichnet, daß man eine den Proteaseinhibitor enthaltende Flüssigkeit, ein synthetisches Peptidsubstrat und die Protease zusammenbringt und zwar so, daß die Protease nicht zugegeben wird, bevor Proteasehinhibitor und Substrat zusammengebracht worden sind, und die Geschwindigkeit der Hydrolyse des Substrats bestimmt.

Dieses Verfahren zur Bestimmung von Proteaseinhibitoren vermeidet eine Reihe der zuvor angeführten Nachteile anderer Bestimmungsverfahren. Das erfindungsgemäße Verfahren kommt ohne Vorinkubation aus und ist für eine ganze Reihe von Enzym-Inhibitorsystemen brauchbar.

Mit diesem Verfahren kann die Konkurrenzreaktion eines Enzyms um einerseits ein chromogenes Substrat und andererseits den zu analysierenden Porteaseinhibitor der Plasma- oder Serumprobe ausgenutzt werden, um fotometrisch eine Information über die Aktivität des Inhibitors zu erhalten. Aufgrund der bei diesem kinetischen Verfahren nicht notwendigen Vorinkubation und Vorverdünnungen wird im Vergleich zu den etablierten Endpunktverfahren nach dem Stand der Technik ein wesentlich höherer Probendurchsatz pro Zeiteinheit erzielt. Außerdem können zumeist auch geringere Mengen an Substrat verwendet werden.

Als Substrat kann besonders ein Tri- oder Tetrapeptid der Formel I

A-B-C-D-R    I

verwendet werden, wobei A der Rest einer Aminosäure in freier oder geschützter Form, eine in der Peptidchemie übliche N-terminale Schutzgruppe oder ein Wasserstoffatom darstellt und B, C und D jeweils der Rest einer freien oder geschützten Aminosäure und R ein chromogener, fluorogener oder luminogener Rest, der durch Fotometrie, Fluorimetrie oder Lumineszenzmessung bestimmt werden kann, oder der Rest eines Mercaptans ist, das nach Hydrolyse der D-R-Bindung mittels einer Reaktion, die einen Farbstoff erzeugt, fotometrisch bestimmt werden kann.

In einer Ausführungsform des Verfahren ist der Inhibitor Antithrombin III und die Protease Thrombin in einer Konzentration von 0,05 bis 1, vorzugsweise 0,3 bis 0,5 U/ml, und das Substrat ein synthetisches Peptidsubstrat, und es wird ein Puffer enthaltend Heparin verwendet, wobei die Konzentration von Heparin im Testansatz 0,02 bis 10 Einheiten/ml, vorzugsweise 2 Einheiten/ml ist.

In einer Ausführungsform ist das Substrat H-D-Phe-Pro-Arg-ANBA-isopropylamid (ANBA = 5-Amino-2-nitrobenzoesäure) und wird in einer Konzentration von 0,1 bis 1 mmol/l, vorzugsweise 0,15 bis 0,25 mmol/l eingesetzt. Es können aber auch andere Substrate, z. B. Tos-Gly-Pro-Arg-para-Nitroanilid, eingesetzt werden.

In einer weiteren Ausführungsform ist die Protease Faktor Xa in einer Konzentration von 0,02 bis 1,0 U/ml, vorzugsweise 0,08 bis 0,12 U/ml, und das Substrat wird in einer Konzentration von 0,1 bis 1 mmol/l, vorzugsweise 0,18 bis 0,25 mmol/l eingesetzt.

In der Ausführungsform mit Faktor Xa ist das Substrat Z-D-Leu-Gly-Arg-2-methoxy-4-nitroanilid oder Benzoyl-Ile-Glu (gamma-Piperidyl)-Gly-Arg-4 nitroanilid.

In weiteren bevorzugten Ausführungsformen können die Protease C1-Esterase, wobei der Inhibitor C1-Inaktivator ist, die Protease Elastase, wobei der Inhibitor alpha$_1$-Antitrypsin, die Protease Chymotrypsin, wobei der Inhibitor alpha$_1$-Antichymotrypsin, und die Protease Kallikrein, wobei der Inhibitor die Kallikreininhibitoren des Plasmas sind, bestimmt werden, wobei ein für die jeweilige Protease geeignetes Substrat benutzt wird.

Es kann eine Puffersubstanz wie Tris, HEPES (N-[2-Hydroxy-ethyl]piperazine-N'-[2-ethansulfonic acid]) oder EPPS (N-[2-Hydroxyethyl]piperazine-N-[2-propanesulfonic acid]) in einer Konzentration von 0 bis 0,5 mol/l, vorzugsweise 0,03 bis 0,1 mol/l, bei einem pH von 7 bis 9, vorzugsweise 7,8 bis 8,2 und ein Neutralsalz, vorzugsweise Kochsalz, in einer Konzentration von 0 bis 0,5 mol/l, vorzugsweise 0,08 bis 0,15mol/l, zugesetzt wird.

Vorteilhaft kann die Geschwindigkeit der Substratspaltung durch Messung der gespaltenen Menge an zwei Zeitpunkten bestimmt werden, von denen der erste bi 0 bis 20, bevorzugt 12 bis 18 sec, der zweite bei 30 bis 300, bevorzugt 80 bis 120 sec nach Zusatz des Enzyms liegt.

Beispiel 1

Bestimmung von Antithrombin III mit einem chromogenen Substrat für Thrombin

a) Eingesetzt wurde Thrombin-Reagenz aus einer Testkombination der Behringwerke (Behrichrom®, AT III). Das Reagenz besteht aus humanem alpha-Thrombin (0,3 IU/ml), Heparin (2,5 USP/ml) in Tris-Puffer pH 8,2. Als Substrat wurde H-D-Phe-Pro-Arg-5-Amino-2-nitrobenzoesäureisopropylamid eingesetzt.
Ansatz: 50 µl Plasma (unverdünnt)
100 µl Substrat (Konz. 4 mMol/l)
1000 µl Thrombin-Reagenz

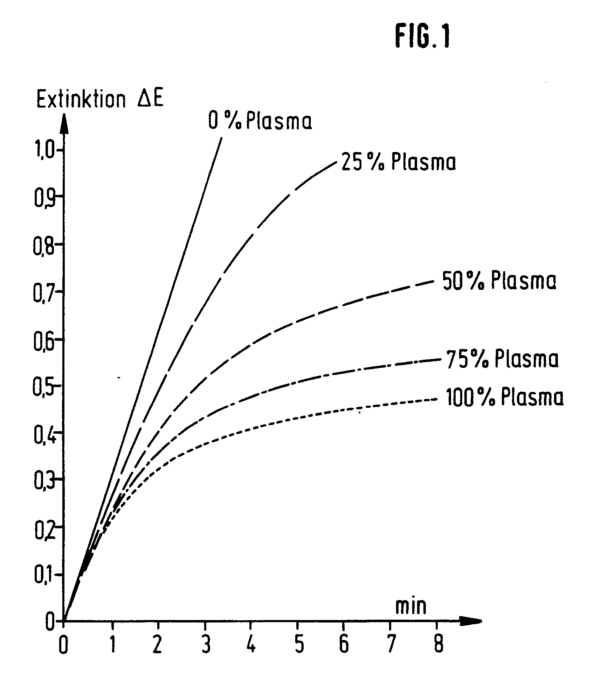

Nach Zusatz des Thrombins wird die Extinktion bei 405 nm auf einem Schreiber registriert. Die Extinktions-Zeitdiagramme verschiedener Plasmakonzentrationen und eines Enzymleerwertes mit Kochsalz anstelle eines Plasma sind in Abbildung 1 gezeigt. Aus diesem Bild kann man ersehen, wie es durch steigende Mengen an Plasma zu einem starken Abknicken der Kurve kommt. Demgegenüber verläuft bei dieser Substratkonzentration der Leerwert zunächst völlig linear, bevor es durch Substratverknappung zu einer Reduktion der Reaktionsgeschwindigkeit kommt.

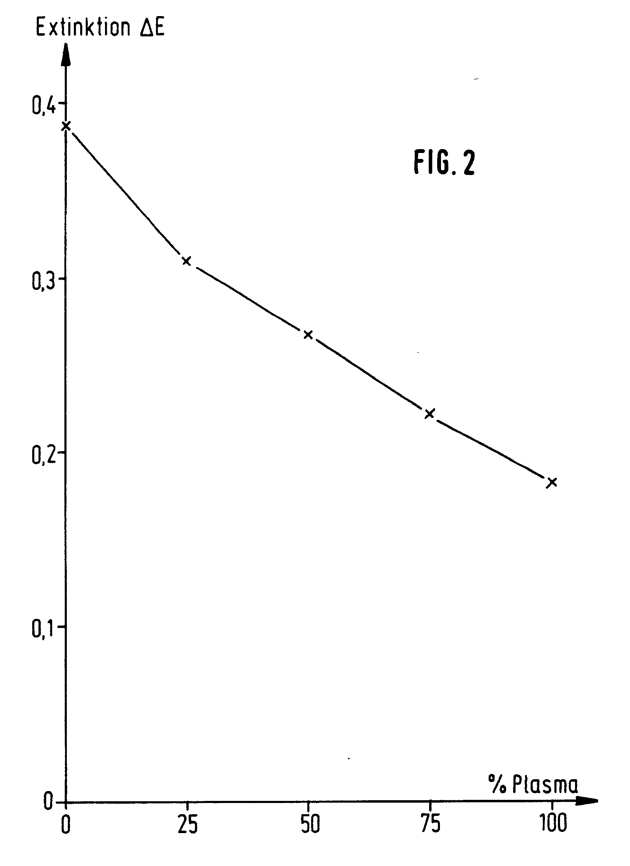

Eine quantitative Aussage über den Gehalt des Plasmas an AT III läßt sich aus diesen Kurven ableiten. Das einfachste und für viele Geräte anwendbare Verfahren besteht in der Messung der Extinktion zu zwei verschiedenen Zeiten, beispielsweise nach 15 und 90 Sekunden. Abbildung 2 zeigt eine Eichkurve, die aus dem oben gezeigten Ansatz für die Extinktionsdifferenz zwischen 90 und 15 Sekunden erhalten wird.

b) Einfluß der Substratkonzentration auf die AT III-Bestimmung

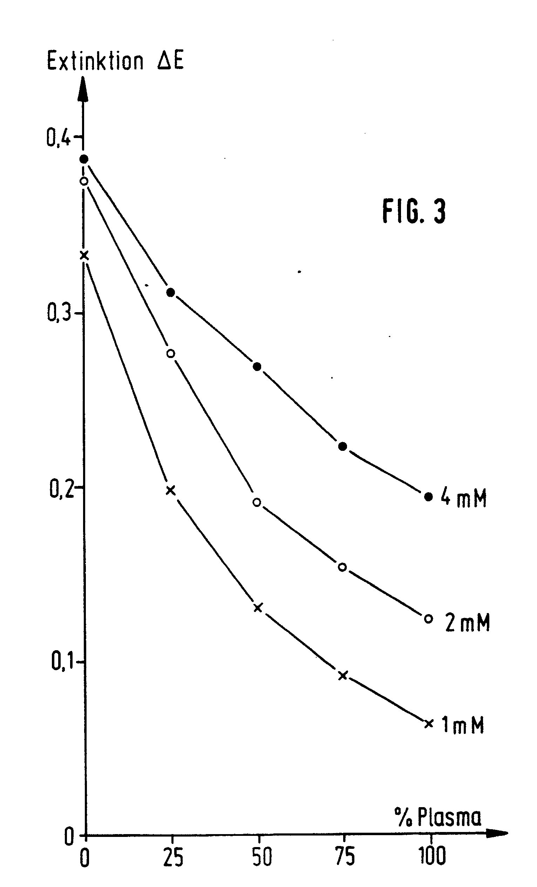

Abbildung 3 zeigt Eichkurven, die bei verschiedenen Substratkonzentrationen erhalten wurden. Daraus geht hervor, daß bei geringer Substratkonzentration die höchste Empfindlichkeit erreicht wird.

c) Methodenvergleich

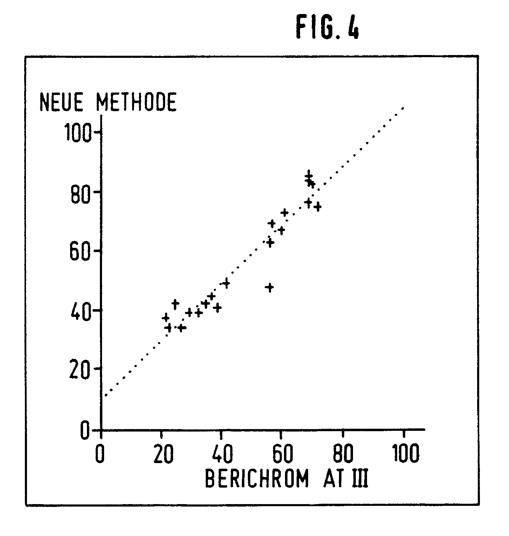

An 20 pathologischen Plasmen wurde die Antithrombin III-Bestimmung in der vereinfachten Form mit einer Bestimmungsmethode nach dem Stand der Technik verglichen. Abbildung 4 zeigt, daß beide Methoden sehr gut übereinstimmen.

Beispiel 2

Bestimmung von Antithrombin III über Faktor Xa

Die Bestimmung von Antithrombin III ist auch über Faktor Xa möglich. Um die Eignung des neuen Verfahrens auch für dieses Enzym zu überprüfen, wurde folgender Ansatz gewählt:

```
   50 µl Substrat (S-2222, Kabi) 3 mM

   50 µl Plasma

 1000 µl Faktor Xa-Reagenz (0,1 U/ml F Xa) in 50 mM TRIS

                                            100 mM NaCl

                                            0,2% Albumin

                                            0,2% PEG 6000

                                            1 E/ml Heparin

                                            pH 8,0: (PEG =

                                            Polyethylen-

                                            glykol)
```

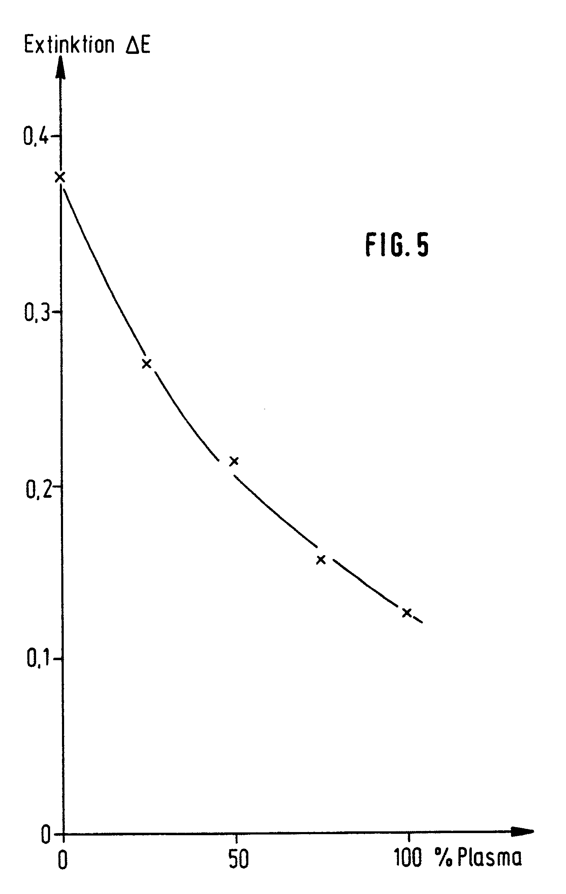

Wie in Beispiel 1 und Abbildung 2 geschildert, erhält man ein ähnliches Extinktions-Zeitdiagramm, aus dem man durch Extinktionsmessung zum Zeitpunkt 15 sec und 105 sec die in Abbildung 5 gezeigte Bezugskurve erstellen kann.

Beispiel 3 Bestimmung von C1-Inaktivator

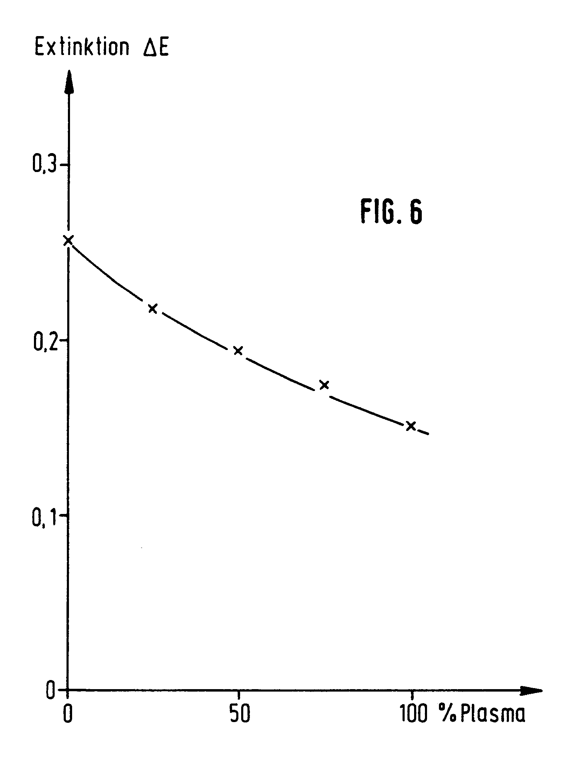

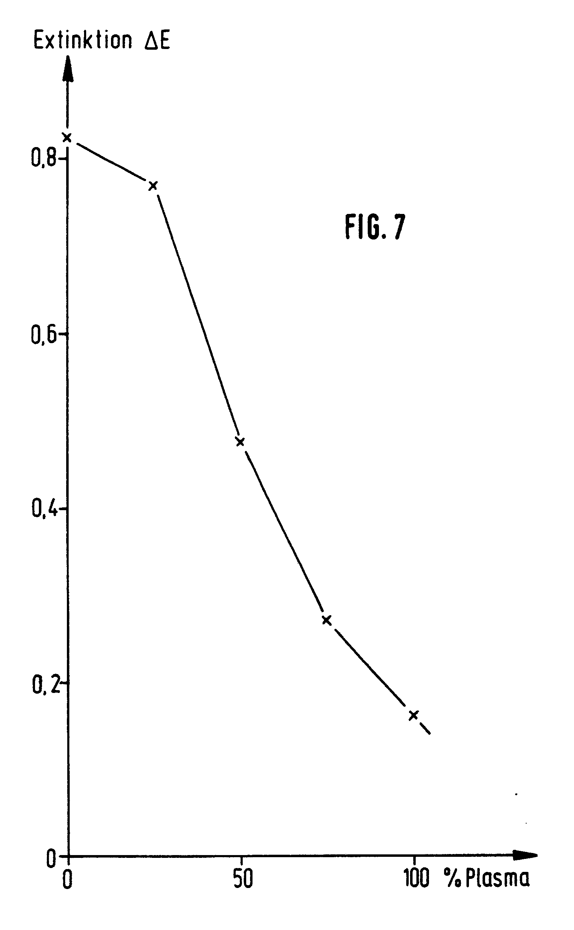

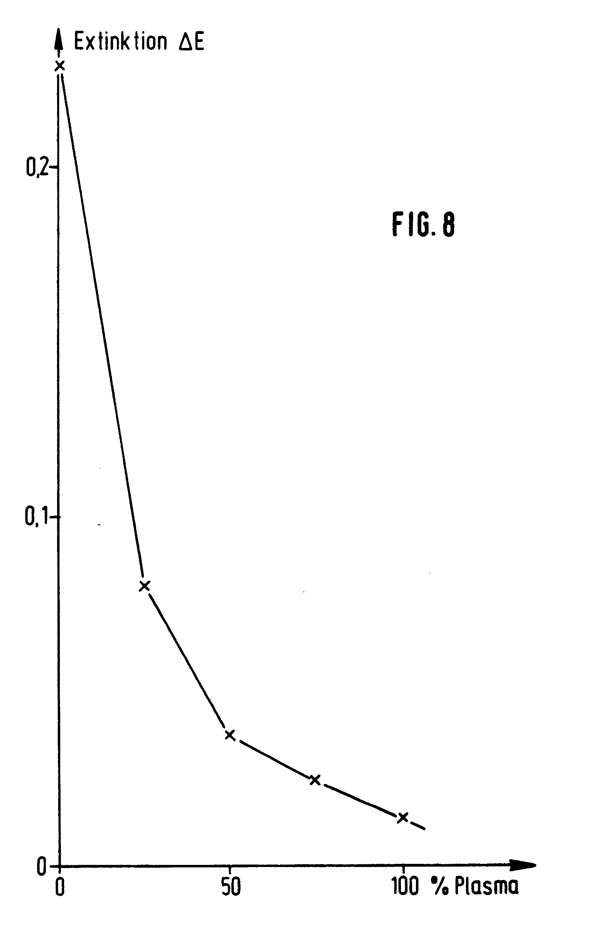

Die Bestimmung von C1-Inaktivator über C1-Esterase mit dem neuen Verfahren wurde wie folgt durchgeführt: 100 µl Plasma
50 µl chromogenes Substrat (N-alpha-Methyloxy-carbonyl
epsilon-Carbobenzoxy-L-Lysyl-glycyl-arginylparanitroanilid), Konzentration 3 mmol/l
1000 µl C1-Esterase (2 ATEE/ml in 100 mM NatriumphosphatPuffer, pH 7,50)
Eine aus der Abbildung 1 analogen Kurvenschar für verschiedene Plasmakonzentrationen hervorgegangene Bezugskurve zeigt Abbildung 6.

Beispiel 4

Bestimmung von alpha$_1$-Antitrypsin
alpha$_1$-Antitrypsin ist der wichtigste Inhibitor des Plasmas für Elastase, die beispielsweise aus Grnaulozyten freigesetzt werden kann. Die Bestimmung des alpha$_1$-Antitrypsins kann durch Hemmung von im Überschuß vorliegender Elastase erfolgen.

Ansatz:
50 µl Plasma
100 µl Substrat (2 mMol/l MeO-Suc-(Ala)$_3$-pNA)

50 μl Esterase (95 E/ml, Sigma)

1000 μl Puffer, 100 mM Tris-Puffer, 150 mM NaCl, pH 8,2

Abbildung 7 zeigt eine Bezugskurve für eine Plasmaverdünnungsreihe, die aus einem Beispiel 1 analogen Auswertung der Kurvenschar erhalten wurde.

Beispiel 5

Bestimmung von alpha₁-Antichymotrypsin

alpha₁-Antichymotrypsin ist ein Plasmainhibitor, der für die Hemmung zellulärer Proteasen verantwortlich ist. Man kann es durch Hemmung von Chymotrypsin bestimmen.

Ansatz:

10 μl Plasma

500 μl Substrat (S-2586, Kabi) 2mM

500 μl Chymotrypsin, 0,001 U/ml in 100 mM TrisPuffer, 150 mM NaCl, pH 8m2)

Abbildung 8 zeigt eine Bezugskurve, die für eine Plasmaverdünnungsreihe erhalten wurde. Die Auswertung erfolgte analog Beispiel 1.

Die gezeigten Beispiele zeigen den Vorteil der erfindungsgemäßen Methode. Bei einem Vergleich, der für eine AT III-Bestimmung notwendigen Zeit, erweist sich beispielsweise das neue Verfahren mit einer Gesamtdauer von etwa 2 min gegenüber den meisten Verfahren des Standes der Technik, die zumeist etwa allein 7 min für die Durchführung der Analyse und noch weitere Zeit für die Probenvorverdünnung erfordern, als wesentlich schneller. Eine Abkürzung der Enzym/Inhibitor-Reaktion ist zwar auch nach Verfahren des Standes der Technik möglich, indem man hohe Dosen des Enzyms vorlegt, jedoch sind dann sehr große Mengen an synthetischen Substraten erforderlich und es werden sehr hohe Reaktionsgeschwindigkeiten erreicht, die man weniger gut messen kann. Der Nachteil einer Probenverdünnung bleibt aber auch hier zumindestens beim AT III, wodurch der Variationskoeffizient der Bestimmung gegenüber anderen Methoden, die einen zusätzliche Verdünnungsschritt nicht erfordern, schlechter ist.

**Patentansprüche**

1.  Verfahren zur Bestimmung eines Proteaseinhibitors mittels einer Protease, die durch diesen Inhibitor gehemmt werden kann, und eines Substrats für diese Protease, dadurch gekennzeichnet, daß man zunächst eine den Proteaseinhibitor enthaltende Flüssigkeit und ein synthetisches Peptidsubstrat zusammenbringt und danach die Reaktion durch Zusatz der Protease startet und die Geschwindigkeit der Hydrolyse des Substrats bestimmt.

2.  Verfahren nach Anspruch 1, dadurch gekennzeichnet, daß das Substrat ein Tri- oder Tetrapeptid der Formel I

    A-B-C-D-R      I

    darstellt, in der A der Rest einer Aminosäure in freier oder geschützter Form, eine in der Peptidchemie übliche N-terminale Schutzgruppe oder ein Wasserstoffatom darstellt und B, C und D jeweils der Rest einer freien oder geschützten Aminosäure und R ein chromogener, fluorogener oder luminogener Rest, der durch Fotometrie, Fluorometrie oder Lumineszenzmessung bestimmt werden kann, oder der Rest eines Mercaptans ist, das nach Hydrolyse der D-R-Bindung mittels einer Reaktion, die einen Farbstoff erzeugt, fotometrische bestimmt werden kann.

3.  Verfahren nach Anspruch 1, dadurch gekennzeichnet, daß der Inhibitor Antithrombin III ist und die Protease Thrombin in einer Konzentration von 0,05 bis 1, vorzugsweise 0,15 bis 0,5 U/ml ist und daß ein Puffer enthaltend Heparin verwendet wird.

4.  Verfahren nach Anspruch 1, dadurch gekennzeichnet, daß eine Puffersubstanz wie Tris, N-[2-Hydroxyäthyl]piperazin-N'-[2-äthansulfonsäure] (HEPES) oder N-[2-Hyroxyäthyl]piperazin-N-[2-propansulfonsäure](EPPS) in einer Konzentration von 0 bis 0,5 mol/l, vorzugsweise 0,03 bis 0,1 mo/l, bei einem pH von 7 bis 9, vorzugsweise 7,8 bis 8,2 und ein Neutralsalz, vorzugsweise Kochsalz, in einer Konzentration von 0 bis 0,5 mol/l, vorzugsweise 0,08 bis 0,15mol/l, zugesetzt wird.

5.  Verfahren nach Anspruch 1, dadurch gekennzeichnet, daß Heparin in einer Konzentration von 0,02 bis 10 Einheiten/ml, vorzugsweise 2 Einheiten/ml zugesetzt wird.

6. Verfahren nach Anspruch 2, dadurch gekennzeichnet, daß das Substrat H-D-Phe-Pro-Arg-ANBA-isopropylamid (ANBA: 5-Amino-2-nitro-benzoesäure) ist und in einer Konzentration von 0,1 bis 1 mmol, vorzugsweise 0,15 bis 0,25 mmol/l eingesetzt wird.

7. Verfahren nach Anspruch 1, dadurch gekennzeichnet, daß die Protease Faktor Xa in einer Konzentration von 0,02 bis 1,0 U/ml, vorzugsweise 0,08 bis 0,12 U/ml, ist und das Substrat in einer Konzentration von 0,1 bis 1 mmol/l, vorzugsweise 0,18 bis 0,25 mmol/l eingesetzt wird.

8. Verfahren nach Anspruch 7, dadurch gekennzeichnet, daß das Substrat Z-D-Leu-Gly-Arg-2-methoxy-4-nitroanilid ist.

9. Verfahren nach Anspruch 1, dadurch gekennzeichnet, daß die Protease C1-Esterase ist und der Inhibitor C1-Inaktivator ist.

10. Verfahren nach Anspruch 1, dadurch gekennzeichnet, daß der Inhibitor alpha$_1$-Antitrypsin und die Protease Elastase ist.

11. Verfahren nach Anspruch 1, dadurch gekennzeichnet, daß der Inhibitor alpha$_1$-Antichymotrypsin und die Protease Chymotrypsin ist.

12. Verfahren nach Anspruch 1, dadurch gekennzeichnet, daß der Inhibitor die Kallikreininhibitoren des Plasmas und die Protease Kallikrein ist.

13. Verfahren nach Anspruch 1, dadurch gekennzeichnet, daß die Geschwindigkeit der Substratspaltung durch Messung der gespaltenen Menge an zwei Zeitpunkten bestimmt wird, von denen der erste bei 0 bis 20, bevorzugt 12 bis 18 sec, der zweite bei 30 bis 300, bevorzugt 80 bis 120 sec nach Zusatz des Enzyms liegt.

## Claims

1. A method for the determination of a protease inhibitor using a protease which can be inhibited by this inhibitor, and using a substrate for this protease, which comprises first mixing a liquid, containing the protease inhibitor, and a synthetic peptide substrate and then starting the reaction by adding the protease, and determining the rate of hydrolysis of the substrate.

2. The method as claimed in claim 1, wherein the substrate represents a tripeptide or tetrapeptide of the formula I

A-B-C-D-R     I

in which A represents the residue of an amino acid in free or protected form, an N-terminal protective group customary in peptide chemistry, or a hydrogen atom, and B, C and D are each the residue of a free or protected amino acid, and R is a chromogenic, fluorogenic or luminogenic radical which can be determined by photometry, fluorimetry or luminescence measurement, or is the radical of a mercaptan which can be determined by photometry after hydrolysis of the D-R bond by a reaction which generates a dyestuff.

3. The method as claimed in claim 1, wherein the inhibitor is antithrombin III, and the protease is thrombin in a concentration of 0.05 to 1, preferably 0.15 to 0.5, U/ml, and wherein use is made of a buffer containing heparin.

4. The method as claimed in claim 1, wherein a buffer substance such as tris, N-[2-hydroxyethyl]-piperazine-N'-[2-ethanesulfonicacid] (HEPES) or N-[2-hydroxyethyl]piperazine-N-[2-propanesulfonic acid] (EPPS) is added in a concentration of 0 to 0.5 mol/l, preferably 0.03 to 0.1 mol/l, at a pH of 7 to 9, preferably 7.8 to 8.2, and a neutral salt, preferably sodium chloride, is added in a concentration of 0 to 0.5 mol/l, preferably 0.08 to 0.15 mol/l.

5. The method as claimed in claim 1, wherein heparin is added in a concentration of 0.02 to 10 units/ml, preferably 2 units/ml.

6. The method as claimed in claim 2, wherein the substrate is the isopropylamide of H-D-Phe-Pro-Arg-ANBA (ANBA: 5-amino-2-nitrobenzoic acid) and is used in a concentration of 0.1 to 1 mmol, preferably 0.15 to 0.25 mmol/l.

7. The method as claimed in claim 1, wherein the protease is factor Xa in a concentration of 0.02 to 1.0 U/ml, preferably 0.08 to 0.12 U/ml, and the substrate is used in a concentration of 0.1 to 1 mmol/l, preferably 0.18 to 0.25 mmol/l.

8. The method as claimed in claim 7, wherein the substrate is the 2-methoxy-4-nitroanilide of Z-D-Leu-Gly-Arg.

9. The method as claimed in claim 1, wherein the protease is Cl esterase, and the inhibitor is Cl inactivator.

10. The method as claimed in claim 1, wherein the inhibitor is $alpha_1$-antitrypsin, and the protease is elastase.

11. The method as claimed in claim 1, wherein the inhibitor is $alpha_1$-antichymotrypsin, and the protease is chymotrypsin.

12. The method as claimed in claim 1, wherein the inhibitor is plasma kallikrein inhibitors and the protease is kallikrein.

13. The method as claimed in claim 1, wherein the rate of substrate cleavage is determined by measurement of the amount cleaved at two points in time, the first being 0 to 20, preferably 12 to 18 sec, and the second being 30 to 300, preferably 80 to 120 sec, after addition of the enzyme.

**Revendications**

1. Procédé pour la détermination d'un inhibiteur de protéase, au moyen d'une protéase qui peut être inhibée par cet inhibiteur et d'un substrat pour cette protéase, caractérisé en ce que l'on met d'abord en contact un liquide contenant l'inhibiteur de protéase et un substrat peptidique synthétique, puis on déclenche la réaction par addition de la protéase et on détermine la vitesse de l'hydrolyse du substrat.

2. Procédé selon la revendication 1, caractérisé en ce que le substrat représente un tri- ou tétrapeptide de formule I

A-B-C-D-R    I

dans laquelle A représente le reste d'un aminoacide sous forme libre ou protégée, un groupe protecteur d'extrémité N-terminale usuel dans la chimie des peptides, ou un atome d'hydrogène, et B, C et D représentent chacun le reste d'un aminoacide libre ou protégé et R représente un radical chromogène, fluorescent ou luminescent, qui peut être dosé par photométrie, fluorimétrie ou mesure de la luminescence, ou le reste d'un mercaptan qui peut être dosé par photométrie, après hydrolyse de la liaison D-R au moyen d'une réaction qui engendre un colorant.

3. Procédé selon la revendication 1, caractérisé en ce que l'inhibiteur est l'antithrombine III et la protéase est la thrombine à une concentration de 0,05 à 1, de préférence de 0,15 à 0,5 U/ml, et en ce que l'on utilise un tampon contenant de l'éparine.

4. Procédé selon la revendication 1, caractérisé en ce que l'on utilise une substance tampon telle que le Tris, l' acide N-(2-hydroxyéthyl)pipérazine-N'-(2-éthanesulfonique) (HEPES) ou l'acide N-(2-hydroxyéthyl)pipérazine-N-(2-propane-sulfonique) (EPPS) à une concentration de 0 à 0,5 mole/l, de préférence de 0,03 à 0,1 mole/l, à un pH de 7 à 9, de préférence de 7,8 à 8,2, et on ajoute un sel neutre, de préférence le chlorure de sodium, à une concentration de 0 à 0,5 mole/l, de préférence de 0,08 à 0,15 mole/l.

5. Procédé selon la revendication 1, caractérisé en ce que l'on ajoute de l'héparine à une concentration de 0,02 à 10 unités/ml, de préférence de 2 unités/ml.

6. Procédé selon la revendication 2, caractérisé en ce que le substrat est le H-D-Phe-Pro-Arg-ANBA-isopropylamide (ANBA: acide 5-amino-2-nitro-benzoïque) et est utilisé à une concentration de 0,1 à 1 mmole, de préférence à 0,15 à 0,25 mmole/l.

7. Procédé selon la revendication 1, caractérisé en ce que la protéase est le facteur Xa à une concentration de 0,02 à 1,0 U/ml, de préférence de 0,08 à 0,12 U/ml, et le substrat est utilisé à une concentration de 0,1 à 1 mmole/l, de préférence de 0,18 à 0,25 mmole/l.

8. Procédé selon la revendication 7, caractérisé en ce que le substrat est le Z-D-Leu-Gly-Arg-2-méthoxy-4-nitroanilide.

9. Procédé selon la revendication 1, caractérisé en ce que la protéase est la Cl-estérase et l'inhibiteur est l'inactivateur de Cl.

10. Procédé selon la revendication 1, caractérisé en ce que l'inhibiteur est l'$\alpha_1$-antitrypsine et la protéase est l'élastase.

11. Procédé selon la revendication 1, caractérisé en ce que l'inhibiteur est l'$\alpha_1$-antichymotrypsine et la protéase est la chymotrypsine.

12. Procédé selon la revendication 1, caractérisé en ce que l'inhibiteur consiste en les inhibiteurs plasmatiques de la kallikréine et la protéase est la kallikréine.

13. Procédé selon la revendication 1, caractérisé en ce que l'on détermine la vitesse de l'hydrolyse du substrat par mesure de la quantité hydrolysée à deux instants, dont le premier se situe à 0-20, de préférence à 12-18 secondes, et le second à 30-100, de préférence à 80-120 secondes après l'addition de l'enzyme.

FIG.1

FIG. 2

FIG. 3

## FIG. 4

FIG. 5

FIG. 6

FIG. 7

FIG. 8